# EUROPEAN PATENT APPLICATION

(11) **EP 4 483 922 A2**
(43) Date of publication of application: **01.01.2025**
(21) Application number: 22928997.0
(22) Date of filing: 23.02.2022
(51) Int. Cl.: A61M 5/145, A61M 5/155, A61M 5/165, A61M 5/168, A61M 5/38, A61J 1/06, A61M 5/14

(54) **MEDICINAL LIQUID PUMPING MODULE AND PREPARING METHOD FOR MEDICINAL LIQUID INJECTION USING SAME**

(71) Applicant: Kim, Yong Hyun, Goyang-si, Gyeonggi-do 10483 (KR)
(72) Inventor: Kim, Yong Hyun, Goyang-si, Gyeonggi-do 10483 (KR)
(74) Representative: Santarelli
(86) International application number: PCT/KR2022/002647
(87) International publication number: WO 2023/163244

(57) **Abstract**

A medicinal liquid pumping module according to an embodiment disclosed herein includes: a chamber having an internal space, which is not filled with a medicinal liquid, and an injection port connected to the internal space; and a plunger disposed inside the chamber such that the internal space has a predetermined volume.

## Description

### TECHNICAL FIELD

The present disclosure relates to a medicinal liquid pumping module and a method of preparing medicinal liquid injection using the same.

### BACKGROUND

In order to supply a medicinal liquid to the patient, a medicinal liquid injection set configured to inject the medicinal liquid (e.g., an injection solution) in a liquid state into a patient is known. Using the medicinal liquid injection set, the medicinal liquid in a predetermined storage space passes through a passage (e.g., the internal spaces of a tube and an injection needle) connected to a patient and is introduced into the body of the patient.

A conventional medicinal liquid injection set injects a medicinal liquid through a process of expansion and contraction. Specifically, when the medicinal liquid is filled into the medicinal liquid injection set, the space filled with the medicinal liquid expands and a pressure is generated to contract the space filled with the medicinal liquid. As a result, the medicinal liquid within the medicinal liquid injection set can be injected into the patient by energy to return the space to its original volume. For example, there is known a medicinal liquid injection set using compressive energy of an elastic body of a spring type, a balloon type, an air compression type or the like or air to generate a pressure for the above-mentioned contraction. In addition, a lubricant for the sliding of the plunger may be applied to the inner surface of the chamber of the medicinal liquid injection set.

In addition, in order to inject a medicinal liquid into a patient's body by using the medicinal liquid injection set, a step of filling the inside of the medicinal liquid injection set with the medicinal liquid is required, and a priming step is required to remove the air inside the tube by filling the tube of the medicinal liquid injection set with a saline solution or a medicinal liquid.

### SUMMARY

E-WHA Biomedics Co., Ltd., a Korean company, has previously sold a product called "ANAPA," a medicinal liquid injection set, and at least some embodiments of the present disclosure present more innovative medicinal liquid injection sets that are upgraded versions of the existing "ANAPA" apparatus.

In the conventional medicinal liquid injection set, while a medicinal liquid is filled into the internal space of the chamber of the medicinal liquid injection set, a plunger slides along the inner surface of the chamber, and the internal space is expanded at the same time. In this process, since the plunger slides along the inner surface of the chamber, the lubricant applied to the inner surface may be damaged or messily deformed. Thereafter, in the process in which the internal space is contracted for injection of the medicinal liquid, when the plunger slides once more along the same inner surface in a direction opposite to the moving direction in the expansion process, frictional resistance acting on the plunger is increased. In addition, it is very difficult to design the frictional resistance because the extent to which the lubricant is damaged or messily deformed cannot be controlled, and the frictional resistance varies depending on which portion of the inner surface the plunger is in contact with during the contraction process. Therefore, the deformation of the lubricant film makes it difficult to set a constant frictional force applied to the plunger during the contraction process, causing a problem in that it is difficult to set a constant flow rate of the medicinal liquid. Embodiments of the liquid medicine pumping apparatus of the present disclosure solve these problems of the prior art.

In addition, the conventional medicinal liquid injection set has a problem in that a very cumbersome operation is required to perform a priming operation. When the priming process is performed in the conventional medicinal liquid injection set by using a pressing actuator (e.g., an air compression type pressing actuator) configured to generate a pressing force during the liquid injection process, there is a problem in that the operation of the pressing actuator has to be started. Embodiments of a method for preparing medicinal liquid injection of the present disclosure solve these problems of the prior art.

In addition, when the plunger of the medicinal liquid injection set is placed at one position in the chamber for a considerable period of time, the inner surface of the chamber may adhere to the plunger. In particular, when a lubricant is applied to the inner surface of the chamber, the adhesion becomes stronger due to the influence of the lubricant. Thus, when the medicinal liquid injection set is used, a pressure higher than a predetermined pressure is necessary to directly move the plunger in the pressing direction for the contraction process. Accordingly, it may be difficult to start the normal operation of the medicinal liquid injection set, and a problem may occur in that the set medicinal liquid injection speed may not be maintained during such an operation start process. Embodiments of the method for preparing medicinal liquid injection of the present disclosure solve this problem.

In order to prevent the above-described deformation of a lubricant film, an aspect of the present disclosure provides embodiments of a medicinal liquid pumping module. A medicinal liquid pumping module according to a representative embodiment includes: a chamber having an internal space, which is not filled with a medicinal liquid, and an injection port connected to the internal space; and a plunger disposed inside the chamber such that the internal space has a predetermined volume.

In an embodiment, an inflow port, which is connected to the internal space and is different from the injection port, may be formed in the chamber. The medicinal liquid pumping module may further include a one-way valve disposed in the inflow port and configured to allow a flow of the medicinal liquid moving from an outside to the internal space and block a flow of the medicinal liquid moving from the internal space to the outside.

In an embodiment, a vent hole may be formed in the chamber. The medicinal liquid pumping module may further include a hydrophobic filter configured to allow passage of air discharged from the internal space to the outside through the vent hole and block passage of a liquid.

In order to solve the problem of the above-described priming operation and the problem of the operation starting failure due to the adhesion of the plunger of the above-described medicinal liquid injection set, another aspect of the present disclosure provides embodiments of a method of preparing medicinal liquid injection. A medicinal liquid injection set used in the method of preparing medicinal liquid injection according to a representative embodiment includes: a chamber having an internal space and having an injection port and an inflow port, which are connected to the internal space and are different from each other; a plunger disposed inside the chamber such that the internal space has a predetermined volume; a pressing actuator configured to be capable of pressing the plunger such that the volume of the internal space is reduced; and a medicinal liquid flow line having a medicinal liquid flow path connected to the injection port. A method of preparing medicinal liquid injection according to a representative embodiment includes: an additional medicinal liquid filling step in which, in a state in which the internal space is filled with a medicinal liquid, additional medicinal liquid is introduced into the internal space through the inflow port such that the plunger moves to increase the volume of the internal space, thereby generating a pressure with which the plunger pushes the medicinal liquid; and a priming step in which the medicinal liquid is moved by the pressure to fill the medicinal liquid flow path.

In an embodiment of the medicinal liquid pumping module, the plunger is disposed inside the chamber such that the internal space which is not filled with the medicinal liquid has a predetermined volume to prevent the plunger from sliding on the inner surface of the chamber when a user's medicinal liquid is filled by the predetermined volume. As a result, damage to the lubricant film applied to the inner surface of the chamber can be minimized. Accordingly, the frictional force applied to the plunger during the above-described contraction process for injecting the medicinal liquid into a patient can be set to be constant, and the medicinal liquid can be injected into the patient at a set constant inflow velocity.

According to an embodiment of the method of preparing medicinal liquid injection, even before the operation of the pressing actuator is initiated, the medicinal liquid additionally introduced into the internal space (the additional inflow amount of the medicinal liquid) by the pressing in the priming step is moved along the connection flow path. Thus, the priming step can be performed quickly and easily. In addition, according to an embodiment of the method of preparing medicinal liquid injection, when a user fills the medicinal liquid of the additional inflow amount into the internal space, a pressure is generated to push the plunger in a direction in which the plunger is to be moved for the priming step without additional manipulation. Thus, the priming step can be conveniently performed with a minimal action.

When the plunger is moved in the pressing direction for the contraction process immediately from the initial stop state, a pressure higher than a set pressure may be required due to the influence of the above-mentioned adhesion. In order to prevent this, in an embodiment of the method of preparing medicinal liquid injection embodiment, the above-described adhesion can be released by first moving the plunger slightly in the opposite direction to the pressing direction in the additional medicinal liquid filling step. Accordingly, when the plunger is pushed in the pressing direction with a predetermined pressure after the additional medicinal liquid filling step, the above-described adhesion is not affected. Thus, the normal operation of the medicinal liquid pumping module or the medicinal liquid injection set can be started naturally, and the medicinal liquid can be injected into a patient at a constant medicinal liquid injection velocity without an additional pressure.

### BRIEF DESCRIPTION OF THE DRAWINGS

FIG. 1 is a conceptual view illustrating an entire system of a medicinal liquid injection set 1 according to an embodiment of the present disclosure.
FIG. 2 is a cross-sectional view of a medicinal liquid pumping module 100 according to a first embodiment.
FIG. 3 is a cross-sectional view of a medicinal liquid pumping module 100' according to a second embodiment.
FIG. 4 is a cross-sectional view of a medicinal liquid pumping module 100" according to a third embodiment.
FIG. 5 is a cross-sectional view of a medicinal liquid pumping module 100‴ according to a fourth embodiment.
FIG. 6 is a cross-sectional view of a medicinal liquid pumping module 100'''' according to a fifth embodiment.
FIG. 7 is a flowchart illustrating a method of preparing medicinal liquid injection according to an embodiment of the present disclosure.
FIG. 8 is a cross-sectional view of the medicinal liquid pumping module 100 illustrating a state in which a medicinal liquid M is being filled into the chamber 110 in the chamber filling step S10 of the preparing method of FIG. 7.
FIG. 9 is a cross-sectional view of the medicinal liquid pumping module 100 illustrating a state in which the filling of the medicinal liquid M into the chamber 110 is completed in the chamber filling step S10 of the preparing method of FIG. 7.
FIG. 10 is a cross-sectional view of the medicinal liquid pumping module 100 illustrating a state in which additional medicinal liquid M is being filled into the chamber 110 in the additional chamber filling step S20 of the preparing method of FIG. 7.
FIG. 11 is a cross-sectional view of the medicinal liquid pumping module 100 illustrating a state in which priming is performed by the medicinal liquid M in the priming step S30 of the preparing method of FIG. 7.

### DETAILED DESCRIPTION

Embodiments of the present disclosure are exemplified for the purpose of explaining the technical idea of the present disclosure. The scope of the rights according to the present disclosure is not limited to the embodiments presented below or the detailed descriptions of such embodiments.

All technical and scientific terms used in the present disclosure have the meaning generally understood by those of ordinary skill in the art to which the present disclosure pertains, unless otherwise defined. All terms used in the present disclosure are chosen for the purpose of more clearly describing the present disclosure and are not chosen to limit the scope of rights according to the present disclosure.

As used in the present disclosure, expressions such as "comprising," "including," "having," and the like are to be understood as open-ended terms having the possibility of encompassing other embodiments, unless otherwise mentioned in the phrase or sentence containing such expressions.

The singular form used in the present disclosure may include a plural meaning unless otherwise mentioned. This equally applies to the singular form recited in the claims.

Terms such as "first" and "second" used in the present disclosure are used in order to distinguish a plurality of components from one another and do not limit the order or importance of the corresponding components.

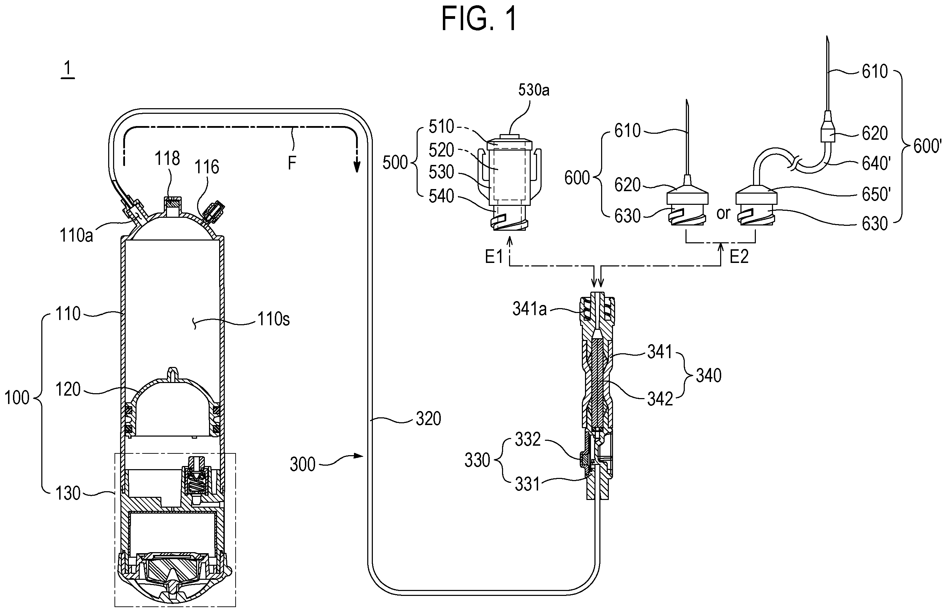

As used in the present disclosure, "upstream" and "downstream" are defined based on the direction in which a medicinal liquid flows when the plunger 120 presses the medicinal liquid. Specifically, the direction of arrow F in FIG. 1 is defined as the downstream direction, and the opposite direction to the downstream direction is defined as the upstream direction.

Hereinafter, embodiments of the present disclosure will be described with reference to the accompanying drawings. In the accompanying drawings, like or relevant components are indicated by like reference numerals. In the following description of embodiments, repeated descriptions of identical or related components may be omitted. However, even if a description of a component is omitted, such a component is not intended to be excluded in an embodiment.

FIG. 1 is a conceptual view illustrating an entire system of a medicinal liquid injection set 1 according to an embodiment of the present disclosure.

Referring to FIG. 1, the medicinal liquid injection set 1 includes a medicinal liquid pumping module 100. The medicinal liquid injection set 1 may include a medicinal liquid flow section 300 connected to the medicinal liquid pumping module 100.

The medicinal liquid pumping module 100 includes a chamber 110 including an internal space 110s which is not filled with a medicinal liquid. An injection port 110a connected to the internal space 110s is formed in the chamber 110. The medicinal liquid flow section 300 may be connected to the injection port 110a. After filling the internal space 110s with the medicinal liquid, the medicinal liquid may be discharged to the medicinal liquid flow section 300 through the injection port 110a.

The medicinal liquid pumping module 100 includes a plunger 120 disposed inside the chamber 110s so that the internal space 110s has a predetermined volume. The plunger 120 is configured to be movable inside the chamber 110. The plunger 120 may press the medicinal liquid inside the chamber 110 by moving in a predetermined pressing direction.

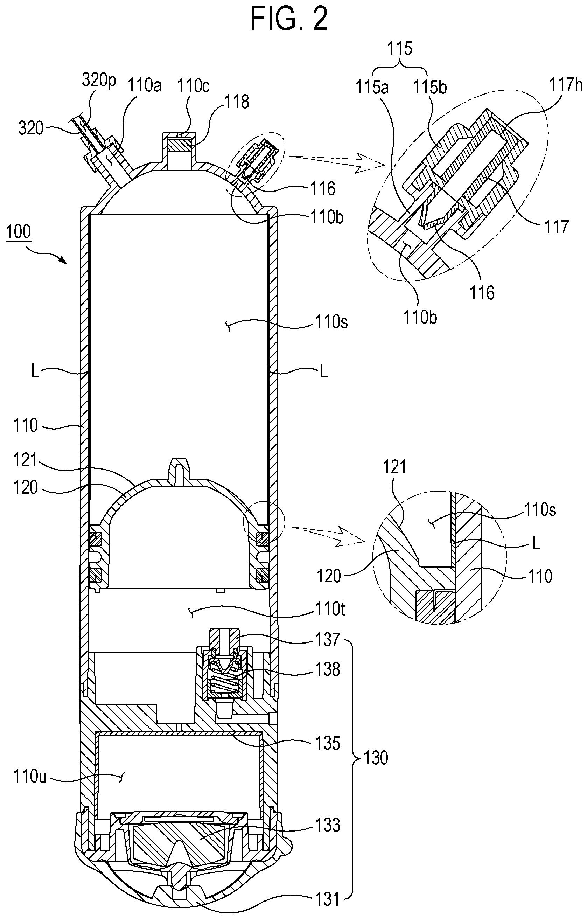

The medicinal liquid pumping module 100 may further include a pressing actuator 130 configured to be capable of pressing the plunger 120 such that the volume of the internal space 110s decreases. For example, the medicinal liquid pumping module 100, 100', 100", or 100‴ according to first to fourth embodiments of the present disclosure includes the pressing actuator 130 *(see* FIGS. 2 to 5). The pressing actuator 130 may provide power such that the plunger 120 moves in the pressing direction. As an example, the pressing actuator 130 may be configured to be capable of pressing the plunger 120 in the pressing direction by a gas pressure generated in the pressing actuator 130 through gas activation, a balloon, or the like. As another example, the pressing actuator 130 may move the plunger 120 in the pressing direction with the force of an elastic body such as a spring. In the present embodiment, the pressing actuator 130 presses the plunger 120 by generating gas (e.g., carbon dioxide) in the pressing actuator 130 by gas activation.

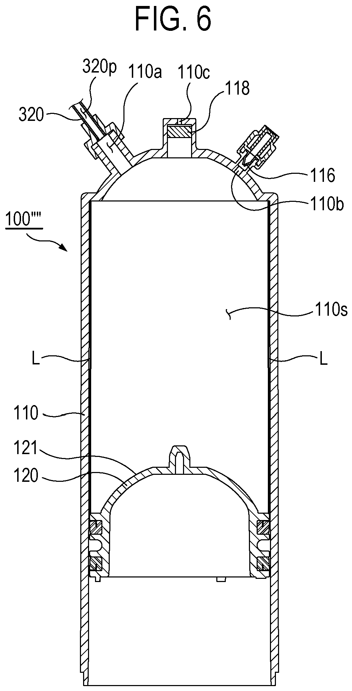

However, the medicinal liquid pumping module may not include the pressing actuator 130. In this case, the medicinal liquid pumping module which does not include the pressing actuator 130 may be manufactured, and users, such as pharmaceutical companies or a medical staff, may couple the pressing actuator 130 to the chamber 110 of the medicinal liquid pumping module by themselves. For example, the medicinal liquid pumping module 100'''' according to a fifth embodiment of the present disclosure does not include the pressing actuator 130 *(see* FIG. 6).

The medicinal liquid flow section 300 includes a medicinal liquid flow line 320. The medicinal liquid flow section 300 may include an air filter 330 and a medicinal liquid transfer tube device 340.

In order to inject the medicinal liquid into a patient by using the medicinal liquid injection set 1, a priming process, which is a process for preparing medicinal liquid injection, and a medicinal liquid injection process may be performed. During the priming process, a priming liquid is allowed to flow along the medicinal liquid flow line 320. In a state in which an end cap 500 is coupled to the downstream end of the medicinal liquid flow section 300, the priming process may proceed. The priming process may be performed in a state in which the patient and the medicinal liquid injection set are separated. The priming liquid flowing along the medicinal liquid flow line 320 is introduced into the air filter 330 and a capillary device for flow rate control 340 (e.g., a filter-integrated medicinal liquid transfer tube device). The medicinal liquid injection set 1 may include an end cap 500 detachably connected to the downstream side of the medicinal liquid transfer tube device 340. The air inside the medicinal liquid flow line 320 may be discharged to the outside through the end cap 500. In the present embodiment, after the medicinal liquid is filled into the medicinal liquid pumping module 100, the medicinal liquid may be used as the priming liquid.

The end cap 500 is configured such that the air passing through the medicinal liquid transfer tube device 340 and the priming liquid flow into the end cap 500. The end cap 500 may be configured to allow air to flow out to the outside but to prevent the priming liquid from flowing out to the outside.

The end cap 500 includes a vent filter 510 configured to block the passage of the priming liquid therethrough but to allow the passage of gas therethrough. The vent filter 510 includes a hydrophobic filter. The end cap 500 may include a sponge 520 disposed upstream of the vent filter 510. The end cap 500 includes an end cap casing 530 configured to accommodate the vent filter 510 in the end cap casing 530. The end cap casing 530 provides a vent hole 530a through which the gas passes. The end cap 500 includes an end cap coupler 540 configured to be capable of being coupled to a downstream connector 341a of the medicinal liquid transfer tube device 340. Arrow E1 in FIG. 1 illustrates the coupling and decoupling directions of the end cap coupler 540 with respect to the downstream connector 341a.

When the end cap 500 is filled with the priming liquid, the end cap 500 may be decoupled from the medicinal liquid transfer tube device 340, and the medicinal liquid transfer tube device 340 and a patient connection unit 600 or 600' may be connected to each other.

The patient connection unit 600 or 600' may include an injection needle 610, a catheter, or the like. The patient connection unit 600 or 600' includes a component which is introduced into a patient's body, such as an injection needle 610.

The patient connection unit 600 or 600' may include an "introduction component including a component to be introduced into the patient's body, such as the injection needle 610" and a "remaining component." The introduction component and the remaining component may be detachably coupled to each other. In this case, in the state in which the introduction component is connected to the patient but is decoupled from the remaining component, the user may couple the remaining component to the medicinal liquid transfer tube device 340 and may then couple the introduction component and the remaining component to each other. In this case, the liquid passing through the medicinal liquid transfer tube device 340 may flow into the patient's body after sequentially passing through the remaining component and the introduction component.

The patient connection unit 600 or 600' includes an injection support 620 configured to support the injection needle 610. The patient connection unit 600 or 600' includes a unit coupler 630 configured to be capable of coupling with a downstream connector 341a of the medicinal liquid transfer tube device 340. Arrow E2 in FIG. 1 illustrates the coupling and decoupling directions of the unit coupler 630 with respect to the downstream connector 341a.

As an example, the patient connection unit 600 may be configured by sequentially connecting the injection needle 610, the injection support 620, and the unit coupler 630.

As another example, the patient connection unit 600' further includes a patient connection tube anchor 650' connected to the downstream side of the unit coupler 630. The patient connection unit 600' further includes a patient connection tube 640' that interconnects the patient connection tube anchor 650' and the injection support 620. The patient connection tube 640' may be formed of a flexible material. The patient connection unit 600' may be configured by sequentially connecting the injection needle 610, the injection support 620, the patient connection tube 640', the patient connection tube anchor 650', and the unit coupler 630.

The medicinal liquid flow line 320 is configured to guide the flow of the priming liquid. The upstream end of the medicinal liquid flow line 320 is connected to the injection port 110a of the chamber 110. For example, the medicinal liquid flow line 320 may be a flexible tube. In a medicinal liquid injection step according to an embodiment, with reference to arrow F, when the plunger 120 is moved in the pressing direction, the medicinal liquid filled into the chamber 110 is capable of passing through the medicinal liquid flow line 320, the air filter 330, and the medicinal liquid transfer tube device 340 in sequence.

The air filter 330 may include a filter casing 331 connected to the medicinal liquid flow line 320 and a filter 332 disposed within the filter casing 331. The filter 332 of the air filter 330 is able to filter out air bubbles.

The medicinal liquid transfer tube device 340 may include a transfer tube casing 341 connected to the air filter 330 and a medicinal liquid transfer tube 342 disposed within the transfer tube casing 341. The medicinal liquid transfer tube 342 may have a capillary flow path configured such that the medicinal liquid flows therethrough. The medicinal liquid transfer tube 342 may have a function of maintaining constant the flow rate per hour of the medicinal liquid flowing along the medicinal liquid flow line 320.

FIG. 2 is a cross-sectional view of the medicinal liquid pumping module 100 according to a first embodiment. The medicinal liquid pumping module 100 will be described in detail with reference to FIG. 2 as follows.

The plunger 120 includes a pressing surface 121 configured to press the medicinal liquid in the internal space 110s. The pressing surface 121 defines one side surface of the internal space 110s. The plunger 120 is disposed inside the chamber 110s so that the internal space 110s has a predetermined volume, wherein the medicinal liquid pumping module 100 is provided such that the predetermined volume is not filled with the medicinal liquid, and the medicinal liquid may be filled into the internal space 110s by a user. Before being filled with the medicinal liquid, the internal space 110s of the medicinal liquid pumping module 100 may be filled with sterilized air.

In the chamber 110, a closed space 110t may be positioned between the plunger 120 and the pressing actuator 130. The pressing actuator 130 may be configured to press the medicinal liquid by the pressure of the gas generated in the pressing actuator 130 through gas activation. The pressure is generated in the closed space 110t on the opposite side to the internal space 110s with reference to the plunger 120 by the pressing actuator 130, and the pressure is maintained constant to press the plunger 120 in the pressing direction, so that the medicinal liquid can be injected into a patient.

In an embodiment, the pressing actuator 130 may include a liquid material (e.g., citric acid) stored in an accommodation space 110u and a solid material 133 (e.g., sodium carbonate) stored to be separated from the liquid material by a partition before the operation of the pressing actuator 130. The pressing actuator 130 may include a pusher 131 configured to be pushable by a user. The pressing actuator 130 may include an air passage filter 135 configured to allow gas generated in the accommodation space 110u to move therethrough to the closed space 110t but to block liquid. The pressing actuator 130 may include a pressure control valve 137 configured to open when the pressure in the closed space 110t exceeds a preset pressure and to connect the closed space 110t to the external space. The pressure control valve 137 may maintain the closed state by the elastic force of the spring 138 when the pressure in the closed space 110t does not exceed the preset pressure.

In an embodiment, when an external force is applied to the pusher 131, the partition is separated and the solid material 133 is introduced into the accommodation space 110u and reacts with the liquid material, whereby a gas (e.g., carbon dioxide) is produced. When the gas generated in the accommodation space 110u passes through the air passage filter 135 and flows into the closed space 110t and the pressure in the closed space 110t increases, the plunger 120 can be moved in the pressing direction.

The medicinal liquid pumping module 100 may include a lubricant L applied to the inner surface of the chamber 110 in which the plunger 120 is slidable while moving. The lubricant L may be applied to the inner surface of the chamber 110 in which the plunger 120 is slidable while moving. The lubricant L may be applied to the inner surface of the chamber 110 defining the internal space 110s which is not filled with the medicinal liquid. For example, the lubricant L may be silicone oil or the like. The lubricant L is applied and cured on the inner surface of the chamber 110 to maintain a resistance value constant when the plunger 120 slides in the pressing direction along the inner surface of the chamber 110, thereby preventing or reducing the occurrence of factors that change the flow velocity of the medicinal liquid. In the process of manufacturing the medicinal liquid pumping module 100, by placing the plunger 120 inside the chamber 110 in such a way that the plunger 120 does not slide on the lubricant L applied to the inner surface, damage or deformation of the applied lubricant L can be minimized during the manufacturing and during the filling of the medicinal liquid by the user.

An injection port 110a connected to the internal space 110s is formed in the chamber 110. The medicinal liquid flow line 320 has a medicinal liquid flow path 320p connected to the injection port 110a.

In the chamber 110, an inflow port 110b connected to the internal space 110s is formed. The medicinal liquid may be filled into the internal space 110s through the inflow port 110b. In the first to third embodiments, the inflow port 110b is different from the injection port 110a, but as in a fourth embodiment to be described later, the inflow port 110b and the injection port 110a may be the same.

The chamber 110 includes an inflow port forming portion 115 that forms the inflow port 110b. The inflow port forming portion 115 may include a first portion 115a fixed to one side of the chamber 110 and a second portion 115b fixed to the first portion 115a. The inflow port 110b may be formed through the first portion 115a and the second portion 115b.

The medicinal liquid pumping module 100 may include a one-way valve 116 disposed in the inflow port 110b. The one-way valve 116 performs the function of a check valve. The one-way valve 116 prevents a reverse flow of the medicinal liquid from the inflow port 110b to the outside of the internal space 110s. The one-way valve 116 allows the flow of the medicinal liquid moving from the outside to the internal space 110s (inflow) but blocks the flow of the medicinal liquid moving from the internal space 110s to the outside (outflow). The one-way valve 116 may include a protrusion (not illustrated) protruding in the direction of the inflow, and a hole (not illustrated) may be formed at a protruding end of the protrusion. The hole of the one-way valve 116 is opened or closed depending on the flow direction of the medicinal liquid in the inflow port 110b. The hole of the one-way valve 116 is opened when the medicinal liquid in the inflow port 110b flows in the inflow direction, and when there is no flow of the medicinal liquid in the inflow port 110b or the medicinal liquid tends to flow in the outflow direction, the hole of the one-way valve 116 is closed. The one-way valve 116 may include a flange (not illustrated) seated by being sandwiched between the first portion 115a and the second portion 115b. The one-way valve 116 may be formed of a flexible material.

The medicinal liquid pumping module 100 may include a pressing valve 117 configured to change whether the inflow port 110b is opened or closed. For example, the pressing valve 117 may be a swabable valve. The second portion 115b may support the pressing valve 117. The pressing valve 117 may include a surface forming a hole 117h which is opened when the pressing valve 117 is pushed from the outside. When a tip of a syringe is pressed from the outside to the surface forming the hole 117h of the pressing valve 117, the surface may be bent to open the hole 117h. The pressing valve 117 may be formed of a flexible material.

A vent hole 110c may be formed in the chamber 110. The vent hole 110c is a separate passage different from the injection port 110a and the inflow port 110b.

The medicinal liquid pumping module 100 may include a hydrophobic filter 118 configured to allow passage of air discharged from the internal space 110s through the vent hole 110c and block the passage of liquid. When a user fills the internal space 110s with a medicinal liquid, air in the internal space 110s may pass through the hydrophobic filter 118 and may be discharged to the outside through the vent hole 110c. Through this, since the user is able to fill the medicinal liquid in the state in which the injection port 110a is blocked, the medicinal liquid injection can be prepared more hygienically.

The hydrophobic filter 118 may be located on the opposite side to the plunger 120 with reference to the internal space 110s. For example, the plunger 120 is located on the lower side and the hydrophobic filter 118 is located on the upper side with reference to the internal space 110s. The chamber 110 may be configured such that the internal space 110s has a shape protruding toward the opposite side (i.e., the opposite side to the plunger with reference to the internal space). The hydrophobic filter 118 may be located in the portion having the protruding shape of the internal space 110s. Through this, the air in the internal space 110s can be efficiently guided to the hydrophobic filter 118 and discharged to the outside.

Although not illustrated, the medicinal liquid pumping module 100 may include a hanger (not illustrated) configured to be hung outside. The hanger may be coupled to a predetermined position of the medicinal liquid pumping module 100 so that the hydrophobic filter 118 is disposed above the internal space 110s by gravity when the hanger is hung outside. For example, the hanger may include a hook, a connector, and the like.

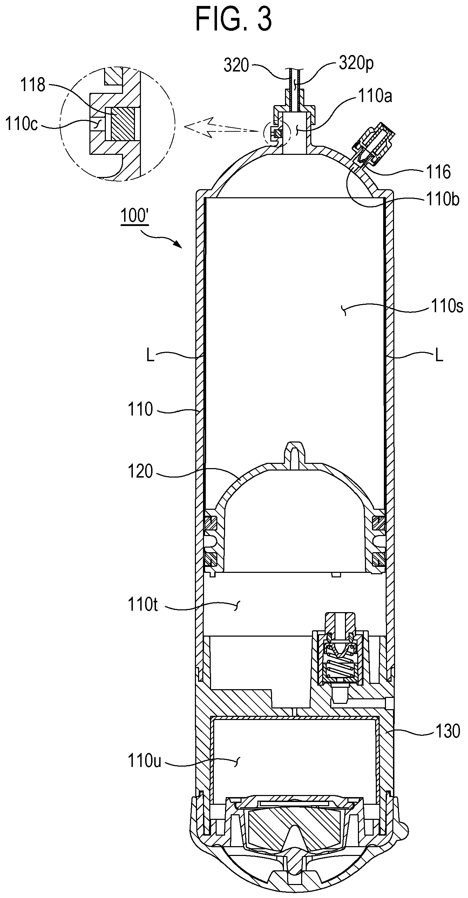

FIG. 3 is a cross-sectional view of a medicinal liquid pumping module 100' according to a second embodiment. Referring to FIG. 3, the medicinal liquid pumping module 100' according to the second embodiment will be described focusing on differences from the medicinal liquid pumping module 100 according to the first embodiment of FIG. 2.

In the medicinal liquid pumping module 100', a vent hole 110c is formed at one side of the injection port 110a. The chamber 110 of the medicinal liquid pumping module 100' may be configured such that the internal space 110s has a shape protruding toward the opposite side (i.e., the opposite side to the plunger with reference to the internal space). An injection port 110a and a hydrophobic filter 118 may be located in the portion having the protruding shape. Through this, the air in the internal space 110s can be efficiently guided to the hydrophobic filter 118 and discharged to the outside.

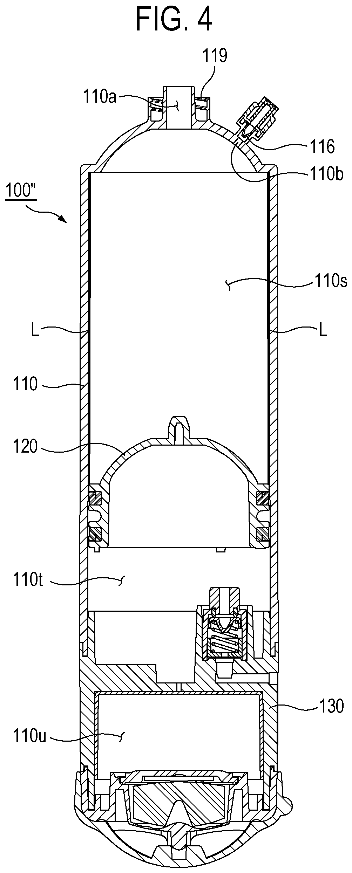

FIG. 4 is a cross-sectional view of a medicinal liquid pumping module 100" according to a third embodiment. Referring to FIG. 4, the medicinal liquid pumping module 100" according to the third embodiment will be described focusing on differences from the medicinal liquid pumping module 100 according to the first embodiment of FIG. 2.

The chamber 110 of the medicinal liquid pumping module 100" does not include the hydrophobic filter 118. In a state in which the injection port 110a is not connected to the medicinal liquid flow line 320, a user may fill a medicinal liquid into the internal space 110s through the inflow port 1 10b, and the air inside the internal space 110s can be discharged to the outside through the injection port 110a. The user may connect the injection port 110a and the medicinal liquid flow line 320 after completing the filling of the medicinal liquid. For example, a screw 119 may be provided to couple the chamber 110 and the medicinal liquid flow line 320 to each other.

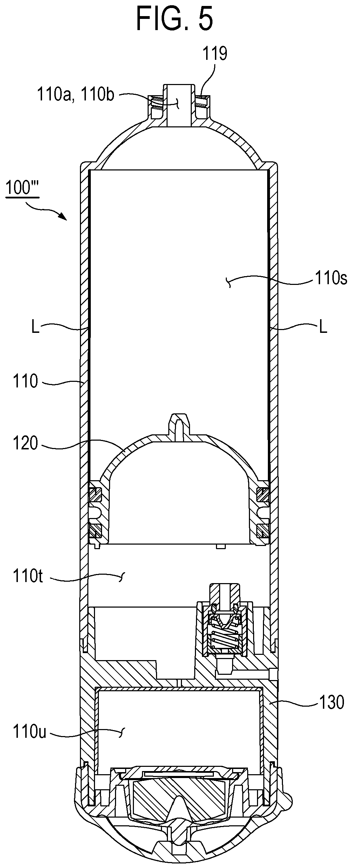

FIG. 5 is a cross-sectional view of a medicinal liquid pumping module 100‴ according to a fourth embodiment. Referring to FIG. 5, the medicinal liquid pumping module 100‴ according to the fourth embodiment will be described focusing on differences from the medicinal liquid pumping module 100" according to the third embodiment of FIG. 4.

The injection port 110a and the inflow port 110b of the medicinal liquid pumping module 100‴ are identical to each other. The medicinal liquid pumping module 100‴ does not include the hydrophobic filter 118. The medicinal liquid pumping module 100‴ does not include the one-way valve 116 and the pressing valve 117. In a state in which the injection port 110a is not connected to the medicinal liquid flow line 320, a user may fill a medicinal liquid into the internal space 110s through the inflow port 110b, which is an injection port 110a, and the air inside the internal space 110s can be discharged to the outside through the inflow port 110b. The user may connect the injection port 110a and the medicinal liquid flow line 320 after completing the filling of the medicinal liquid. For example, a screw 119 may be provided to couple the chamber 110 and the medicinal liquid flow line 320 to each other.

FIG. 6 is a cross-sectional view of a medicinal liquid pumping module 100'''' according to a fifth embodiment. As described above, the medicinal liquid pumping module which includes the pressing actuator 130 coupled to the chamber 110 may be manufactured *(see* FIGS. 2 to 5), or medicinal liquid pumping module 100'''' which does not include the pressing actuator 130 may be manufactured *(see* FIG. 6). FIG. 6 illustrates the medicinal liquid pumping module with the pressing actuator 130 removed from the medicinal liquid pumping module according to FIG. 2, however the medicinal liquid pumping module with the pressing actuator 130 removed from the medicinal liquid pumping module according to any one of FIGS. 3 to 5 may also be implemented. In the case of the medicinal liquid pumping module 100'''' which does not include the pressing actuator 130, users can couple a separate pressing actuator 130 to the chamber 110 by themselves so that the above-mentioned closed space 110t is formed.

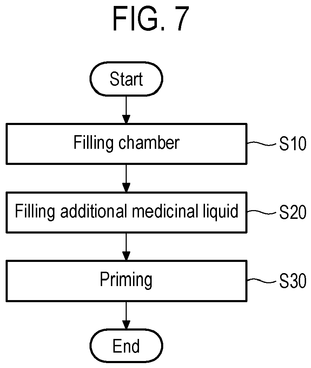

FIG. 7 is a flowchart illustrating a method of preparing medicinal liquid injection according to an embodiment of the present disclosure. The method for preparing medicinal liquid injection may be a method of using a medicinal liquid injection set including a chamber 110, in which an internal space 100s is formed and in which an injection port 110a and an inflow port 110b, which are different from each other and are connected to the internal space 100s, are formed. Hereinafter, with reference to FIGS. 7 to 11, the method of preparing medicinal liquid injection will be described in detail with reference to the medicinal liquid injection set 1 including the medicinal liquid pumping module 100 according to the first and fifth embodiments. However, the method of preparing medicinal liquid injection may be performed by using the medicinal liquid injection set including the medicinal liquid pumping module 100' or 100" according to the second and third embodiments including an inflow port 110b, which is different from the injection port 110a.

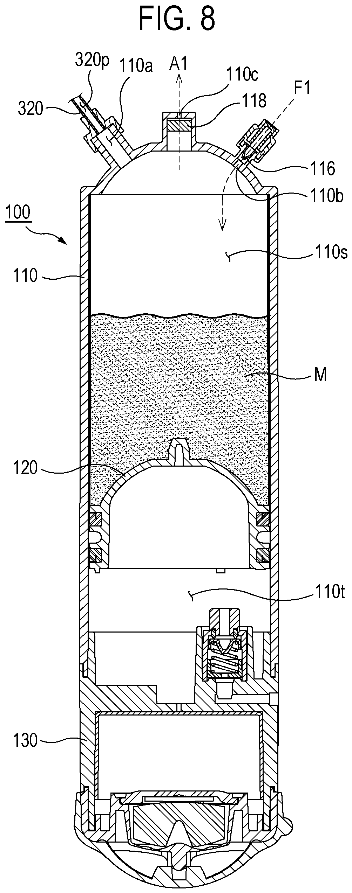

FIG. 8 is a cross-sectional view of the medicinal liquid pumping module 100 illustrating a state in which a medicinal liquid M is being filled into the chamber 110 in the chamber filling step S10 of the preparing method of FIG. 7. Referring to FIGS. 7 and 8, the preparing method may include a chamber filling step S10 of filling the medicinal liquid M into the internal space 110s of the chamber 110. In the chamber filling step S10, the medicinal liquid M can be introduced into the internal space 110s of the chamber 110 through the inflow port 110b *(see* arrow F1), and the air in the internal space 110s can be discharged to the outside via the hydrophobic filter 118 and through the vent hole 110c (*see* arrow A1). According to an embodiment, the chamber filling step S10 may be performed by a user before proceeding with medicinal liquid injection in a hospital or the like, but the chamber filling step S10 may be performed in the process of manufacturing a pre-filled medicinal liquid pumping module 100 by a user who manufactures the pre-filled medicinal liquid pumping module 100.

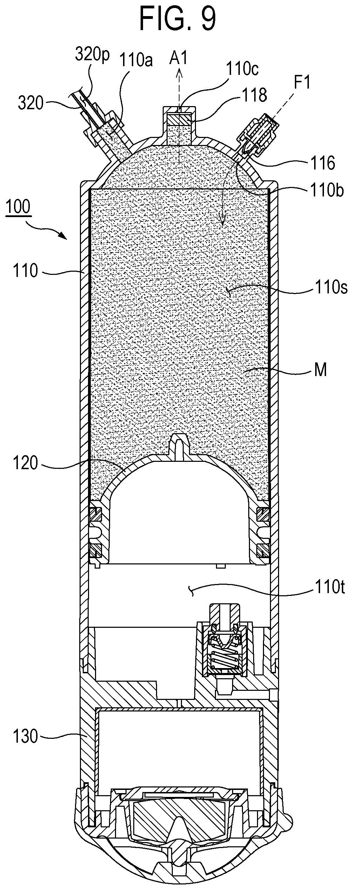

FIG. 9 is a cross-sectional view of the medicinal liquid pumping module 100 illustrating a state in which the filling of the medicinal liquid M into the chamber 110 is completed in the chamber filling step S10 of the preparing method of FIG. 7. Referring to FIG. 9, when the chamber filling step S10 is completed, the internal space 110s of the chamber 110 is filled with the medicinal liquid M.

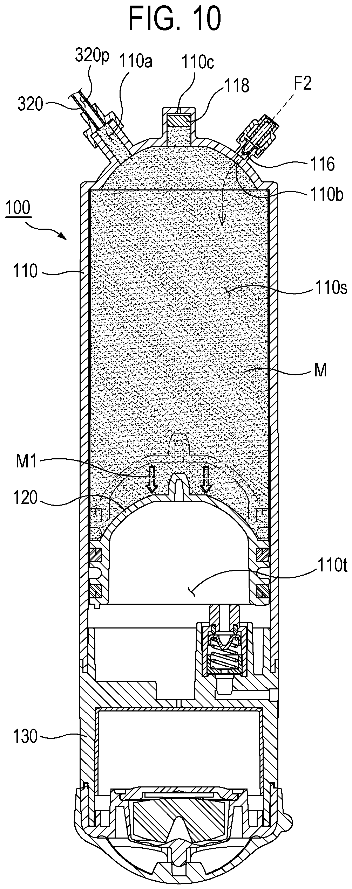

FIG. 10 is a cross-sectional view of the medicinal liquid pumping module 100 illustrating a state in which additional medicinal liquid M is being filled into the chamber 110 in the additional chamber filling step S20 of the preparing method of FIG. 7. Referring to FIGS. 7 and 10, the preparing method includes the additional medicinal liquid filling step S20 in which, in a state in which the internal space 110s is filled with the medicinal liquid M, additional medicinal liquid M is introduced into the internal space 110s through the inflow port 110b, so that the plunger 120 is moved to increase the volume of the internal space 110s (*see* arrow M1), whereby a pressure of pushing the medicinal liquid M by the plunger 120 is generated. In the additional medicinal liquid filling step S20, the additional medicinal liquid M can flow into the internal space 110s through the inflow port 110b (*see* arrow F2).

Here, the pressure with which the plunger 120 pushes the medicinal liquid M may be a pressure that increases in the closed space 110t. In the additional medicinal liquid filling step S20, the pressure may be generated by compression of the closed space 110t on the opposite side to the internal space 110s with reference to the plunger 120. The plunger 120 is moved in the direction M1 opposite to the pressing direction by the medicinal liquid M added to the internal space 110s so that the closed space 110t can be compressed.

The amount of the medicinal liquid M filled in the internal space 110s before the additional medicinal liquid filling step S20 (*see* the amount of the medicinal liquid illustrated in FIG. 9) is greater than the amount of the medicinal liquid M filled in the additional medicinal liquid filling step S20. The amount of the medicinal liquid M filled in the additional medicinal liquid filling step S20 may be the amount of the medicinal liquid to fill the flow path of the medicinal liquid flow section 300 such as the medicinal liquid flow path 320p (i.e., the amount of the medicinal liquid required for priming).

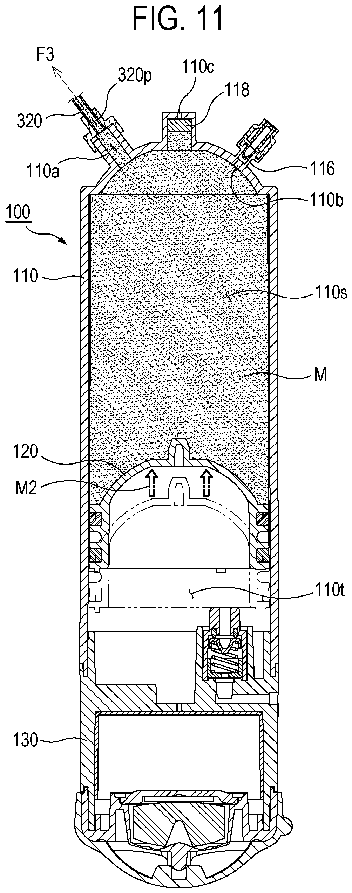

FIG. 11 is a cross-sectional view of the medicinal liquid pumping module 100 illustrating a state in which priming is performed by the medicinal liquid M in the priming step S30 of the preparing method of FIG. 7. Referring to FIGS. 6 and 10, the preparing method includes the priming step S30 in which the medicinal liquid M is moved by the pressure, which is generated in the additional medicinal liquid filling step S20, and with which the plunger 120 pushes the medicinal liquid M, to fill the medicinal liquid flow path 320p. The priming step S30 may be initiated after the additional medicinal liquid filling step S20 is initiated first and before the additional medicinal liquid filling step S20 is terminated.

The technical idea of the present disclosure has been described heretofore with reference to some embodiments and examples illustrated in the accompanying drawings. However, it is to be understood that various substitutions, modifications, and alterations may be made without departing from the technical idea and scope of the present disclosure that can be understood by those of ordinary skill in the technical field to which the present disclosure pertains. In addition, it is to be understood that such substitutions, modifications, and alterations fall within the scope of the appended claims.

## Claims

1. A medicinal liquid pumping module comprising:
a chamber having an internal space, which is not filled with a medicinal liquid, and an injection port connected to the internal space; and
a plunger disposed inside the chamber such that the internal space has a predetermined volume.

2. The medicinal liquid pumping module of Claim 1, further comprising:
a lubricant applied to an inner surface of the chamber in which the plunger is configured to be slidable while moving.

3. The medicinal liquid pumping module of Claim 1, wherein an inflow port, which is connected to the internal space and is different from the injection port, is formed in the chamber, and
wherein the medicinal liquid pumping module further comprises:
a one-way valve disposed in the inflow port and configured to allow a flow of the medicinal liquid moving from an outside to the internal space and block a flow of the medicinal liquid moving from the internal space to the outside.

4. The medicinal liquid pumping module of Claim 3, wherein a vent hole is formed in the chamber, and
wherein the medicinal liquid pumping module further comprises:
a hydrophobic filter configured to allow passage of air discharged from the internal space to the outside through the vent hole and block passage of a liquid.

5. The medicinal liquid pumping module of Claim 1, wherein a vent hole is formed in the chamber, and
wherein the medicinal liquid pumping module further comprises:
a hydrophobic filter configured to allow passage of air discharged from the internal space to the outside through the vent hole and block passage of a liquid.

6. The medicinal liquid pumping module of Claim 5, wherein the hydrophobic filter is located on an opposite side to the plunger with reference to the internal space.

7. The medicinal liquid pumping module of Claim 6, wherein the chamber is configured such that the internal space has a shape protruding toward the opposite side, and the hydrophobic filter is located in a portion having the protruding shape of the internal space.

8. The medicinal liquid pumping module of Claim 1, further comprising:
a pressing actuator configured to be capable of pressing the plunger such that the volume of the internal space is reduced,
wherein the pressing actuator is configured to be capable of pressing the plunger by a pressure of a gas generated in the pressing actuator.

9. A preparation method for medicinal liquid injection using a medicinal liquid injection set comprising a chamber having an internal space and having an injection port and an inflow port, which are connected to the internal space and are different from each other; a plunger disposed inside the chamber such that the internal space has a predetermined volume; a pressing actuator configured to be capable of pressing the plunger such that the volume of the internal space is reduced; and a medicinal liquid flow line having a medicinal liquid flow path connected to the injection port, the method comprising:
an additional medicinal liquid filling step in which, in a state in which the internal space is filled with a medicinal liquid, additional medicinal liquid is introduced into the internal space through the inflow port such that the plunger moves to increase the volume of the internal space, thereby generating a pressure with which the plunger pushes the medicinal liquid; and
a priming step in which the medicinal liquid is moved by the pressure to fill the medicinal liquid flow path.

10. The preparation method of Claim 9, wherein, in the additional medicinal liquid filling step, the pressure is generated by compressing a closed space on an opposite side to the internal space with reference to the plunger.

11. The preparation method of Claim 9, wherein an amount of the medicinal liquid filled in the internal space before the additional medicinal liquid filling step is greater than an amount of the medicinal liquid filled in the additional medicinal liquid filling step.

12. The preparation method of Claim 9, wherein the medicinal liquid injection set further comprises a lubricant applied to an inner surface of the chamber in which the plunger is configured to be slidable while moving.
